# EUROPÄISCHE PATENTANMELDUNG

(11) **EP 0 984 268 A2**
(43) Veröffentlichungstag der Anmeldung: **08.03.2000**
(21) Anmeldenummer: 99117073.9
(22) Anmeldetag: 31.08.1999
(51) Int. Cl.: G01N 21/62, C12M 1/34

(54) **Verfahren zur Erfassung ultraschwacher Photonenemission und dessen Verwendung**

(30) Priorität: 03.09.1998 DE 19840100
(71) Anmelder: Beiersdorf Aktiengesellschaft, 20245 Hamburg (DE)
(72) Erfinder: Mei, Weiping, Dr., 20245 Hamburg (DE); Sauermann, Gerhard, Dr., 24649 Wiemersdorf (DE); Hoppe, Udo, Prof. Dr., 24598 Heidmühlen (DE)

(57) **Zusammenfassung**

Gegenstand der Erfindung ist ein Verfahren zur Erfassung ultraschwacher Photonenemission (UPE) von Gewebeoberflächen, dadurch gekennzeichnet, daß die ultraschwache Photonenemission von Oberflächen von Zellkulturen oder von Gewebeoberflächen oder Organoberflächen von Menschen oder Tieren zweidimensional erfaßt wird und dazu auch gegebenenfalls der zeitliche Verlauf der Intensität dieser Emissionen auf diesen Oberflächen erfaßt wird und dessen Verwendung.

## Beschreibung

Gegenstand der Erfindung ist ein Verfahren zur Erfassung von ultraschwacher Photonenemission und dessen Verwendung, insbesondere zur Erfassung des physiologischen Zustandes der Haut, z.B. menschlicher Haut.

Bisher wurde die Messung 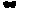 ultraschwacher Photonenemission" (Licht im Spektralbereich von 200 bis 800 nm, in der Größenordnung von 10⁻⁹ bis 10⁻⁷ Lux, entsprechend einigen bis zu tausend Photonen (s⁻¹ cm⁻²)) von menschlicher Haut auf die Intensität beschränkt . So entstanden ausnahmslos Meßgeräte, die mit einem gekühlten Photomultiplier und weiteren bekannten Bauelementen (wie z. B. Vorverstärker, Verstärker, Diskriminator, Kühlung, Spannungsversorgung und Datenerfassung) ausgestattet waren. Für diese Messung mußte ein Abstand zum Objekt und damit ein bestimmtes Meßvolumen eingehalten werden. Ein solches Meßverfahren wurde in dem deutschen Gebrauchsmuster G 94 17 845.3 zur Erfassung 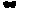 ultraschwacher Photonenemission" (im folgenden auch 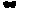 UPE" genannt) von biologischen Proben und menschlicher Haut beschrieben. Eine zweidimensionale, die Meßfläche punktweise erfassende, und zeitliche Erfassung dieser Meßwerte war damit jedoch nicht möglich.

Aus der Literatur ist weiterhin ein System zur Erfassung der Entwicklung der flächenmäßigen und zeitlichen Darstellung der UPE von Sojasprossen bekannt. (Kobayshi M, Devaraj B, Usa M, Tanno Y, Takeda M, Inaba H, 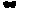 Development and applicaiton of new technology for two-dimensional space-time characterization and correlation analysis of ultraweak biophoton information." Frontiers Med. Biol. Engng.; 1996; 7:229-309).

Das vom Objekt ausgehende Licht wird dabei über ein Linsensystem auf den Meßkopf übertragen. Messungen an Haut und Haaren sind nicht beschrieben.

Aufgabe der Erfindung war es, ein Verfahren zur zweidimensionalen, zeitlichen Erfassung von UPE, von Gewebeoberflächen oder Organoberflächen, insbesondere von Hautoberflächen zu schaffen.

Erfindungsgemäß werden die Nachteile des Standes der Technik überwunden.

Gegenstand der Erfindung ist ein Verfahren zur Erfassung ultraschwacher Photonenemission (UPE) von Gewebeoberflächen, dadurch gekennzeichnet, daß die ultraschwache Photonenemission von Oberflächen von Zellkulturen oder von Gewebeoberflächen oder Organoberflächen von Menschen oder Tieren zweidimensional erfaßt wird und dazu auch gegebenenfalls der zeitliche Verlauf der Intensität dieser Emissionen auf diesen Oberflächen erfaßt wird.

Mit der zweidimensionalen Erfassung der UPE wird die Lage, bzw. der Ort, einer Vielzahl einzelner Meßpunkte oder Meßbereiche, d.h. Photonen, auf einer bestimmten Fläche erfaßt und damit die örtliche Auflösung erreicht. Mit einem Photomultiplier wurde bisher nur die Meßfläche insgesamt erfaßt. Der Entstehungsort des Photons konnte damit nicht erkannt. werden.

Die erfindungsgemäßen Oberflächen können in vivo oder in vitro vorliegen. Bevorzugt werden Haut oder Hautanhangsgebilde wie Haare oder Nägel untersucht, insbesondere menschliche Haut oder Hautanhangsgebilde.

Vorzugsweise werden Photonen im Bereich von 250 nm bis 700 nm gemessen, also im UV- und sichtbaren Licht-Bereich.

Der Vorteil des Verfahrens gemäß der Erfindung beruht darauf, daß eine Erfassung der ultraschwachen Photonenemission insbesondere von menschlicher Haut in vivo, in zweidimensionaler Form erfolgt und daß neben der Erfassung der Ortsinformation auch gleichzeitig die Zeitinformation von jedem Photon erfaßt werden kann. durch diese neue Form der Erfassung oder Messung ist es möglich, eine detaillierte Kinetikbeschreibung über die zeitlichen Vorgänge und örtliche Korrelation direkt und gleichzeitig zu dokumentieren.

Gegenstand der Erfindung ist auch eine Vorrichtung, die die Ausführung des vorstehenden Verfahrens gestattet.

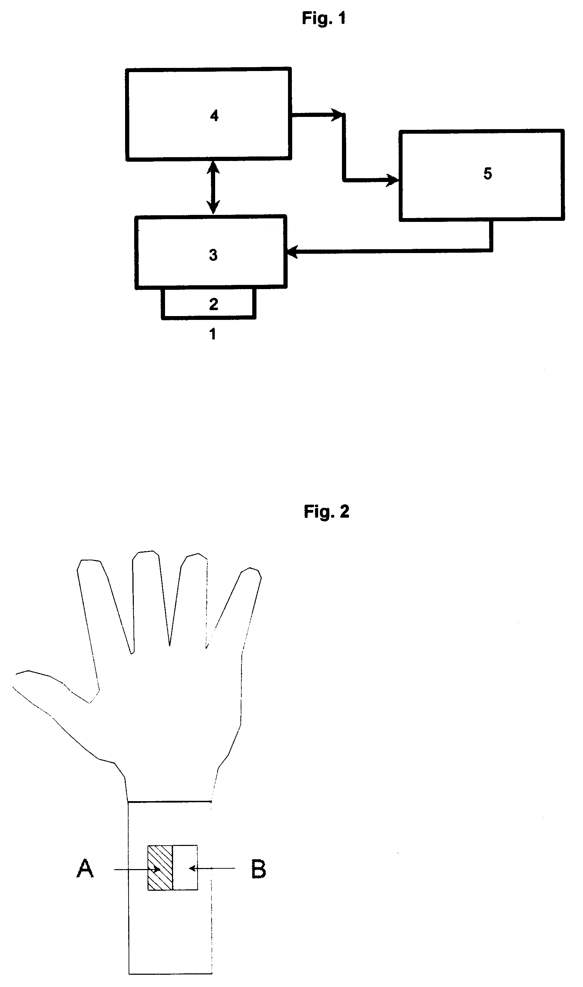

Ein Schema ist in Fig. 1 abgebildet.

Von einer Probe (1) werden die zu messenden Photonen vorzugsweise mit einem Adapter (2) zu einem 2D-Meßkopf geleitet. Diese gesamte Anordnung befindet sich vorzugsweise in einem Dunkelraum. Ein Computer (4) mit Steuerungskarte und einem Programm zur Erfassung und Rekonstruktion der Orts- und Zeitinformation ist mit dem Meßkopf verbunden. Die Stromversorgung, ein Steuerungsgerät und die Prozeßsteuerung können in einer Einheit (5) zusammengefaßt sein.
Als Probe (1) kann beispielsweise die lebende Haut am Unterarm verwendet werden. Es ist in der Praxis vorteilhaft, die Probe nicht direkt in Kontakt mit der Kathode des Meßkopfes zu bringen, sondern einen Adapter (2) zu verwenden, beispielsweise eine Faser-Optik, die mit einem Ende auf der Haut aufliegt und am anderen Ende mit dem Meßkopf (3) verbunden ist. Ein geeigneter Adapter wird z.B. vom Hersteller des 2D-Meßkopfes (Photek Ltd.) angeboten.

Der 2D-Meßkopf, geeignet für zweidimensionale Messungen, enthält z.B. eine Photonen-Zähl-Kamera (Hersteller Photek Ltd., East Sussex, UK), die mit dem Adapter verbunden ist. die Kamera arbeitet vorzugsweise mit mindestens drei MCPs (Multichannel-Plates (z.B. 25mm 3 stage multichannel plate), die mit der CCD-Kamera (z.B. FTM 800, Hersteller Philips) verbunden sind, z.B. durch eine Faser-Optik. Die auf einem Bildschirm entstehende Abbildung wird von der CCD-Kamera abgelesen.

Die zu vermessende Oberfläche (1) bzw. Probe, der Adapter (2) und der Meßkopf (3) werden vorzugsweise in einem klimatisierten Dunkelraum untergebracht.

Vorzugsweise arbeitet das System z.B. ohne Kühlung bei niedrigem Dark-Noise von z.B. weniger als 40 cps (counts per second) bei 23°C und erreicht bei einem aktiven Areal von 3 cm² eine Bildauflösung von 752 x 570 Pixel. Dies entspricht einem Pixel von 25 Mikron². Die CCD-Kamera liefert eine Folge von 25 Bildern pro Sekunde, ist mit Standard-TV-Monitoren und Videorecordern kompatibel und gestattet die Echt-Zeit-Beobachtung der Messungen.

Für die Computer-Auswertung werden z.B. eine Peak Processor PC-Card und die IFS-216 Software benutzt (Fa. Photek).

Die verwendeten Geräte der Vorrichtung sind bekannt und im Handel erhältlich.

Mit dem erfindungsgemäßen Verfahren ist es möglich, den physiologischen Zustand der erfindungsgemäßen Oberfläche, insbesondere der menschlichen Haut, in vivo und in vitro zu erfassen. Durch den optischen Adapter (2) ist der Kontakt zwischen Sensor und Oberfläche gewährleistet, so daß eine zweidimensionale ultraschwache Photonenemission im Zeitablauf darstellbar ist. Dabei liegt der Adapter auf der Oberfläche auf und berührt sie also.

Das erfindungsgemäße Verfahren kann z.B. zur Charakterisierung von unterschiedlichen physiologischen Zuständen der vorstehend genannten Oberflächen verwendet werden, die sich z.B. bei unterschiedlichen Produktanwendungen, insbesondere von topischen Zubereitungen wie Dermatika oder Kosmetika, oder Zuständen, die sich z.B. vor oder nach UV-Bestrahlung ergeben, beispielsweise nach Produktanwendungen oder unterschiedlichen Produktanwendungen.

Auch Wirkungen von oral verabreichten Wirkstoffen können untersucht werden, insbesondere in der oben beschriebenen Weise.

Gegenstand der Erfindung ist daher auch die Verwendung des erfindungsgemäßen Verfahrens zur Charakterisierung des physiologischen Zustandes von Oberflächen von Zellkulturen oder von Gewebeoberflächen oder Organoberflächen von Menschen oder Tieren, insbesondere zum Vergleich von unterschiedlichen Behandlungszuständen dieser Oberflächen.

Besonders geeignet sind physiologische Zustände, an denen Oxidationsvorgänge beteiligt sind oder beteiligt waren.

Beispielsweise kann das erfindungsgemäße Verfahren dazu verwendet werden, den Ort oxidativ gestreßter Haut, z.B. nach Anwendungen von Benzoylperoxid, zu ermitteln, insbesondere in Verbindung mit oder z.B. nach der Anwendung von Produkten oder Wirkstoffen.

In Fig. 2,3 und 4 sind Ergebnisse von Messungen dargestellt.

Fig. 2 zeigt ein markiertes Areal auf dem Unterarm. Eine Hälfte davon bleibt unbehandelt, die andere Hälfte wurde mit einem Sonnenschutzmittel behandelt. Der Unterarm wird dann natürlichem Sonnenlicht oder der Strahlung eines Sonnensimulators (300W, L.O.T-Oriel GmbH, DE) ausgesetzt, z.B. 30s lang. Anschließend wird die UPE-Messung durchgeführt.

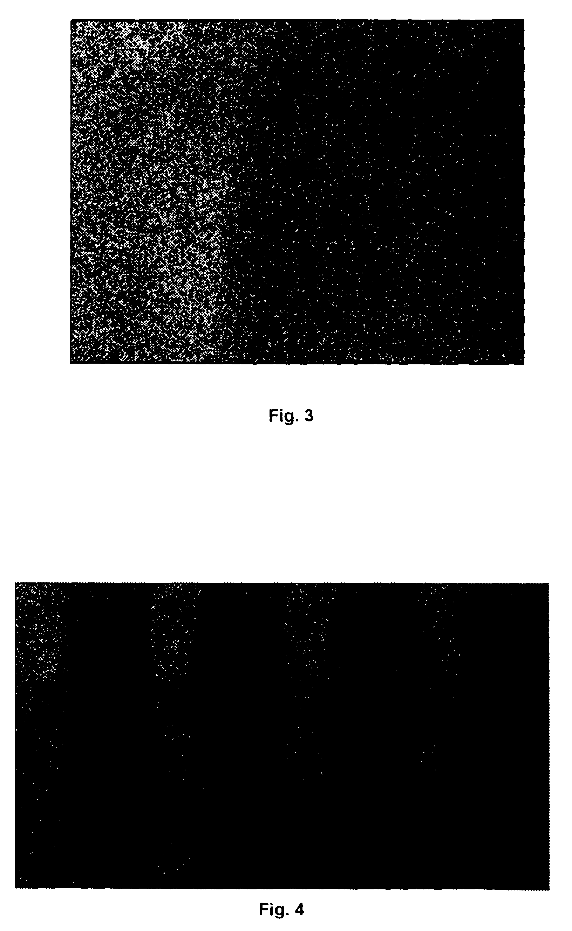

Man erhält ein Meßergebnis wie in Fig. 3 dargestellt. Es existiert ein deutlicher Unterschied zwischen behandelter (B) und unbehandelter (A) Haut. Die ungeschützte Haut zeigt wesentlich mehr UV-induzierte UPE als die geschützte Haut. Unterschiedliche Wirkungsstärke verschiedener Kosmetika oder Sonnenschutzmittel lassen sich direkt auf einem Bild bei gleichzeitiger Anwendung auf den beiden Hälften des Meßareales vergleichen oder durch eine zweite, nachfolgende Messung. Die Aufnahmezeit kann etwa 5 min betragen.

Fig. 4 zeigt eine Folge von Meßbildern. Aus den gesamten Bildern oder Meßdaten kann die Software eine Zeitreihenfolge erstellen. Daraus wird die Zeitinformation über die zeitliche Entwicklung der Lichtemission ermittelt. In Fig. 4 wird die Kinetik des Abklingen der Photonenemission nach 30s UV-Bestrahlung auf 10 Bildern von je 10s Belichtungszeit, die aufeinander folgen, dargestellt.

Unter den obengenannten Bedingungen kann eine Reihe von Bildern (in Abhängigkeit von der Zeit) eines Hautareals in beliebige Zeitintervalle aufgeteilt werden. Fig. 4 zeigt das typische Ergebnis. Die Unterschiede in der Photonenemission zwischen linker und rechter Seite bestehen darin, daß auf der rechten Sehe ein Lichtschutzprodukt verwendet wurde.

Überraschenderweise zeigte sich auch, daß mit Hilfe dieser neuen Methode eine eindeutige Zuordnung des Ortes von oxidativ gestreßter Haut (z.B. durch Anwendung von Benzoylperoxid) durch ultraschwache Photonenemission möglich ist.

Die Meßergebnisse zeigen, daß bereits ein einziges Photon als einzelner Lichtpunkt abgebildet werden kann. Die zweidimensionale Information kann auf dem Bild direkt gezeigt werden und auch die Kinetik dieser Prozesse läßt sich als zeitlich gestaffelte Bilderfolge einfach darstellen, auch in Form einer Intensität / Zeit Funktion. Der örtliche und zeitliche Ablauf einer UPE kann einfach und genau sogar für unterschiedliche Oberflächen und z.B. Behandlungen dieser Oberflächen sogar gleichzeitig verglichen und aufgezeichnet werden.

Das erfindungsgemäße Verfahren kann insbesondere für eine Vielzahl von Untersuchungen auf dem Gebiet der Kosmetik, Dermatologie oder Hautforschung verwendet werden, beispielsweise für die Untersuchung der Wirksamkeit von Antioxidantien und UV-Filtern, insbesondere in topischen Zubereitungen, dem Schutz vor insbesondere UV-induzierter Oxidation oder anderen Prozessen mit Beteiligung freier Radikale.

Auch zur Untersuchung von Tumorzellkulturen oder Oberflächen mit Tumorzellen oder von Tumorgewebe ist das erfindungsgemäße Verfahren hervorragend geeignet.

## Patentansprüche

1. Verfahren zur Erfassung ultraschwacher Photonenemission (UPE) von Gewebeoberflächen, dadurch gekennzeichnet, daß die ultraschwache Photonenemission von Oberflächen von Zellkulturen oder von Gewebeoberflächen oder Organoberflächen von Menschen oder Tieren zweidimensional erfaßt wird und dazu auch gegebenenfalls der zeitliche Verlauf der Intensität dieser Emissionen auf diesen Oberflächen erfaßt wird.

2. Verfahren gemäß Anspruch 1, dadurch gekennzeichnet, daß Photonen im UV- und sichtbaren Licht-Bereich gemessen werden.

3. Verfahren gemäß Anspruch 1, dadurch gekennzeichnet, daß die Oberflächen, insbesondere Oberflächen der Haut oder von Haut- oder Hautanhangsgebilden, in vivo oder in vitro erfaßt werden.

4. Verfahren gemäß Anspruch 1, dadurch gekennzeichnet, daß ein optischer Adapter bei der Messung verwendet wird, der auf der zu messenden Oberfläche aufliegt, d.h. sie berührt.

5. Verwendung des Verfahrens gemäß Anspruch 1 zur Charakterisierung des physiologischen Zustandes von Oberflächen von Zellkulturen oder von Gewebeoberflächen oder Organoberflächen von Menschen oder Tieren, insbesondere zum Vergleich von unterschiedlichen Behandlungszuständen dieser Oberflächen.
